# EUROPEAN PATENT APPLICATION

(11) **EP 4 603 093 A1**
(43) Date of publication of application: **20.08.2025**
(21) Application number: 23877700.7
(22) Date of filing: 12.10.2023
(51) Int. Cl.: A61K 31/454, A61P 13/12, A61P 29/00

(54) **PHARMACEUTICAL COMPOSITION FOR PREVENTING OR TREATING NEPHRITIS**

(30) Priority: 12.10.2022 KR 20220130547
(71) Applicant: Daewoong Pharmaceutical Co., Ltd., Hwaseong-si, Gyenoggi-do 18623 (KR)
(72) Inventor: LEE, Caroline Hee, Seoul 06170 (KR); CHO, Min Jae, Seoul 06170 (KR); PARK, Joon Seok, Seoul 06170 (KR)
(74) Representative: Germain Maureau
(86) International application number: PCT/KR2023/015710
(87) International publication number: WO 2024/080777

(57) **Abstract**

The present invention provides a pharmaceutical composition that can be usefully used for the prevention or treatment of nephritis.

## Description

### [TECHNICAL FIELD]

The present invention provides a pharmaceutical composition for preventing or treating nephritis.

### [BACKGROUND ART]

Diseases that cause inflammation in the kidneys are broadly called nephritis or kidney inflammation, and the cause of inflammation can include inflammation caused by bacteria and inflammation caused by an immune response. Depending on the sites of inflammation, it is divided into Tubulointerstitial Nephritis (TIN), Acute/Chronic pyelonephritis, and Glomerulonephritis. In addition, it can also occur as a complication of other diseases, and may be divided into diabetic nephropathy, lupus nephritis, and the like depending on the cause. Such nephritis can lead to complications of chronic renal failure, and it is difficult to recover kidney function at the time of developing into chronic renal failure, and thus, appropriate treatment is necessary in the early stages.

Tubulointerstitial nephritis refers to a pathological condition where inflammatory cells infiltrate into the renal tubules and interstitium, resulting in decreased kidney function. Tubulointerstitial nephritis is induced by drug therapy, systemic diseases (e.g., systemic lupus erythematosus (SLE)) and infections. Severely reduced urine output (oliguria) appears in 50% of patients with acute tubulointerstitial nephritis, and gross hematuria appears in 5% of patients, and may be accompanied by joint pain, fever, blood eosinophilia, and skin rash, but symptoms may not appear. If the symptoms of tubulointerstitial nephritis appear and BUN and creatinine levels increase due to decreased renal function in a blood tests, tubulointerstitial nephritis is diagnosed, and steroids are used for treatment.

The glomerulus is a tissue made of capillary clump that is the basic unit for filtering blood in the kidneys. Glomerulonephritis refers to a disease in which symptoms and signs occur when an inflammatory response occurs in the glomerulus due to immune dysfunction. The causes of glomerulonephritis are very diverse, but are mainly caused by immunological mechanisms. Glomerulonephritis is diagnosed through urine tests, blood tests, and kidney tissue tests, and immunosuppressants are mainly used as therapeutic agents.

On the other hand, PRS (prolyl-tRNA synthetase) is one of the aminoacyl-tRNA synthetase (ARS) family enzymes, and plays a role in activating amino acids for protein synthesis. That is, ARS performs a translational function which forms aminoacyl adenylate (AA-AMP) and then moves the activated amino acid to the 3rd terminus of the corresponding tRNA. Because ARS plays a key role in protein synthesis, ARS inhibition inhibits the growth and development of all cells. Accordingly, ARS is recognized as a promising target for antibiotics or disease therapeutic agents which have to inhibit overexpression of cells (Nature, 2013, 494: 121-125).

PRS exists and functions as a multi-synthetase complex (MSC) state in the form of EPRS (Glutamyl-Prolyl-tRNA Synthetase). In particular, it has been reported that among various MSCs, EPRS functions as a translational silencer that inhibits the production of VEGF A (vascular endothelial growth factor A), which is a key factor in angiogenesis, and is also closely related to various solid cancers (Nat. Rev. Cancer, 2011, 11, 708-718).

Therefore, the present inventors have carried out intensive studies on methods for preventing or treating nephritis, and as a result, found that a specific PRS inhibitor, which will be described later, can be used for preventing or treating nephritis, and completed the present invention.

### [DETAILED DESCRIPTION OF THE INVENTION]

### [Technical Problem]

It is an object of the present invention to provide a pharmaceutical composition that can be usefully used for the prevention or treatment of nephritis.

### [Technical Solution]

In order to achieve the above object, according to the present invention, there is provided a pharmaceutical composition for preventing or treating nephritis, comprising a compound represented by the following Chemical Formula 1, or a pharmaceutically acceptable salt thereof:

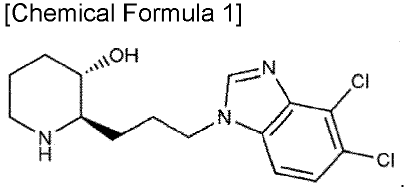

The compound represented by Chemical Formula 1 or a pharmaceutically acceptable salt thereof is a compound described in Korean Patent Registration No. 10-2084772, and is specifically a substance described in Example 40 of that specification. It is known that the above substance is a PRS inhibitor and can be used for the prevention or treatment of fibrosis.

The present invention has confirmed that the above substance can also be applied to the prevention or treatment of nephritis, and as confirmed in Examples and Experimental Examples described below, it can be usefully used for the prevention or treatment of nephritis.

On the other hand, the compounds represented by Chemical Formula 1 may be respectively used in the form of a pharmaceutically acceptable salt, and as the salt, an acid addition salt formed by a pharmaceutically acceptable free acid is useful. As the free acid, an inorganic acid and an organic acid may be used. Examples of the inorganic acid may include hydrochloric acid, bromic acid, sulfuric acid, phosphoric acid, and the like. Examples of the organic acid may include citric acid, acetic acid, lactic acid, maleic acid, gluconic acid, methanesulfonic acid, succinic acid, 4-toluene sulfonic acid, glutamic acid, aspartic acid or the like. Preferably, the pharmaceutically acceptable salt of the compound represented by Chemical Formula 1 is hydrochloride.

Further, the compound represented by Chemical Formula 1 can be prepared in crystalline form or non-crystalline form. When the compound represented by Chemical Formula 1 is prepared in crystalline form, it may be optionally hydrated or solvated. The present invention may include not only stoichiometric hydrates of the compound represented by Chemical Formula 1 but also compounds containing a various amount of water. The solvates of the compound represented by Chemical Formula 1 include both stoichiometric solvates and non-stoichiometric solvates.

Meanwhile, examples of the nephritis include tubulointerstitial nephritis, glomerulonephritis, acute pyelonephritis, or chronic pyelonephritis.

As used herein, the term "prevention" refers to any act to delay or inhibit occurrence, spread or recurrence of the above-mentioned diseases by administration of the composition of the present invention, and "treatment" refers to any act to improve or change the symptoms of the above diseases for the better by administration of the composition of the present invention. Specifically, the pharmaceutical composition according to the present invention has an effect of improving renal function. Additionally, the pharmaceutical composition according to the present invention has the effect of reducing inflammatory cells CD4+, CD8+, or gdT in kidney tissue.

The pharmaceutical composition according to the present invention can be formulated in types for oral or parenteral administrations according to a standard pharmaceutical practice. These formulations may contain additives such as pharmaceutically acceptable carrier, adjuvant or diluent in addition to the active ingredient.

Suitable carriers include, for example, physiological saline, polyethylene glycol, ethanol, vegetable oil, and isopropyl myristate and the like. Diluents include, for example, lactose, dextrose, sucrose, mannitol, sorbitol, cellulose and/or glycine and the like. but are not limited thereto. Further, the compounds of the present invention can be dissolved in oils, propylene glycol or other solvents commonly used in the preparation of injection solutions. Furthermore, the compounds of the present invention can be formulated in ointments or creams for topical application.

In pharmaceutical dosage forms, the compounds of the present invention may be used in the form of pharmaceutically acceptable salts or solvates thereof, and they may also be used alone or in appropriate association, as well as in combination, with other pharmaceutically active compounds. In one example, the pharmaceutical composition according to the present invention may further include other active components used for the prevention or treatment of nephritis. Additionally, when the pharmaceutical composition according to the present invention further comprises other active component, the weight ratio of the compound represented by Chemical Formula 1, or a pharmaceutically acceptable salt thereof, and the other active component is preferably 1:0.1 to 1:10.

The compounds of the present invention can be formulated into injection solutions by dissolving, suspending or emulsifying the compounds in a water-soluble solvent such as normal saline, 5% dextrose or a non-aqueous solvent such as synthetic fatty acid glyceride, higher fatty acid ester or propylene glycol. Formulations of the present invention may include conventional additives such as solubilizers, isotonic agents, suspending agents, emulsifying agents, stabilizers and preservatives.

The pharmaceutical composition according to the present invention may be administered through oral or parenteral routes. Depending on the method of administration, the composition according to the present invention may contain the compound represented by Chemical Formula 1, or a pharmaceutically acceptable salt thereof in an amount of 0.001 to 99% by weight, preferably 0.01 to 60% by weight.

The pharmaceutical composition according to the present invention may be administered to mammals such as a rat, a mouse, a domestic animal, a human, through various routes. The administration may be carried out through all possible methods, for example, oral, rectal, intravenous, intramuscular, subcutaneous, intra-endometrial, intracerebroventricular injection.

### [Advantageous Effects]

The pharmaceutical composition according to the present invention can be usefully used for the prevention or treatment of nephritis.

### [BRIEF DESCRIPTION OF THE DRAWINGS]

FIG. 1 schematically shows the process of isolating normal human blood using a centrifuge in Experimental Example 1 of the present invention.
FIG. 2 shows the results of cell proliferation inhibition in CD4+ and CD8+ T cells by the compound of the present invention in Experimental Example 1 of the present invention.
FIG. 3 shows the results of IL-17 cytokine inhibition in gdT cells by the compound of the present invention in Experimental Example 2 of the present invention.
FIG. 4 shows the results of measuring blood BUN and Creatinine which are primary indicators of kidney function by treatment with the compound of the present invention in a mouse renal failure treatment model using an Adenine diet in Experimental Example 3 of the present invention.
FIG. 5 shows the results of measuring the degree of fibrosis through immunochemical staining of mouse kidney tissue after treatment with the compound of the present invention in a mouse renal failure treatment model using an Adenine diet in Experimental Example 3 of the present invention.
FIG. 6 shows the results of inhibition of fibrotic factors after treating a rat kidney cell line with the compounds of the present invention in Experimental Example 3 of the present invention.
FIG. 7 shows the results of inhibition of fibrotic factors after treating human primary kidney cells with the compound of the present invention in Experimental Example 4 of the present invention.
FIG. 8 shows the results of measuring blood BUN and Creatinine, which are primary indicators of kidney function by treatment with the compound of the present invention in a mouse renal failure prevention model using an Adenine diet in Experimental Example 5 of the present invention.
FIG. 9 shows the results of measuring the degree of collagen accumulation in kidney tissue after treatment with the compound of the present invention in a mouse renal failure prevention model using an Adenine diet in Experimental Example 5 of the present invention.
FIG. 10 shows the results of confirming the effect on damaged glomerular tissue after treating an NTN model with the compound of the present invention in Experimental Example 6 of the present invention.
FIG. 11 shows the results of confirming the effect that appeared on damaged glomerular tissue after treating the NTN model with the compound of the present invention through immunochemical staining of mouse kidney tissue in Experimental Example 6 of the present invention.

### [DETAILED DESCRIPTION OF THE EMBODIMENTS]

Hereinafter, the present invention will be explained in more detail with reference to the following examples. However, the following examples are for illustrative purposed only, and are not intended to limit the scope of the present invention in any way.

### Example

The following compound was prepared in the same manner as in Example 40 of Korean Patent Registration No. 10-2084772, and was named "PRSi" or "Example" hereinafter.

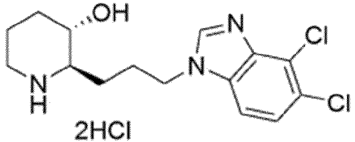

¹H NMR (500 MHz, MeOD): δ 9.67 (s, 1H). 8.02 (d, 1H), 7.82 (d, 1H), 4.62 (m, 2H), 3.60 (m, 1H), 3.28 (m, 1H), 2.99 (m, 2H), 2.25 (m, 2H), 2.08 (m, 2H), 1.99 (m, 1H), 1.78 (m, 2H), 1.54 (m, 1H)

### Experimental Example 1: CD4+, CD8+ T cell proliferation inhibition efficacy

Through the Adenine diet animal model, it was confirmed that CD4+, CD8+, and gdT cells (IL-17) are the main factors in the renal failure model. Thus, the effect of the compound of the present invention on the above major factors was confirmed through Experimental Examples 1 and 2 of the present invention.

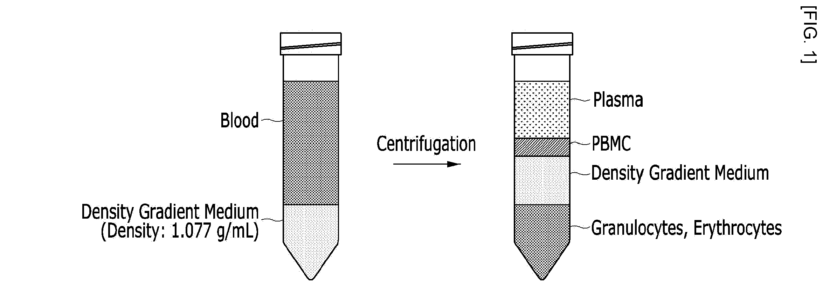

After 10 ml of normal human blood was collected, as shown in FIG. 1, ficoll density gradient medium (Cytiva 17144003) and PBS were added, and then the immune cell layer (PBMC) was isolated using a centrifuge.

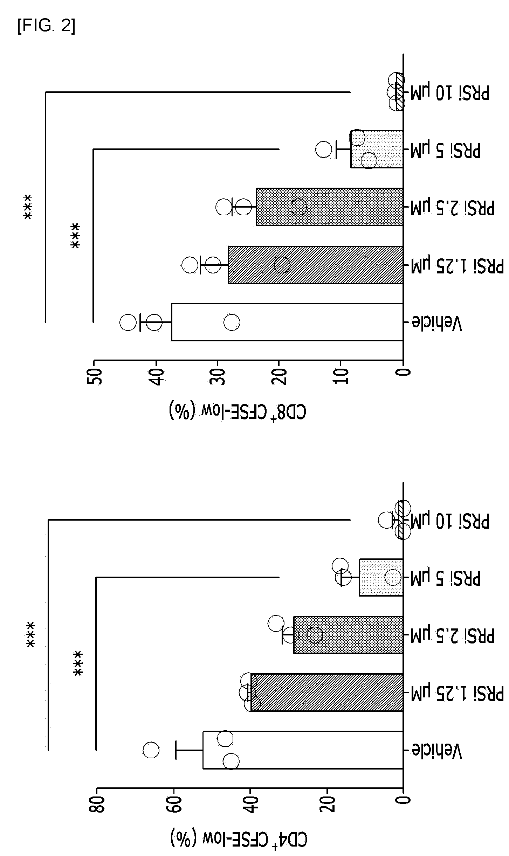

PBMCs were transferred to a new tube using a pipette. After RBC lysis buffer was added to PBMC, centrifugation was performed, and then CD8 or CD4 cells were isolated using CD8 MicroBeads (Miltenyl Biotec, 130-045-201) or CD4 MicroBeads (Miltenyl Biotec, 130-096-533). A CFSE (-(and-6)-Carboxyfluorescein Diacetate, Succinimidyl Ester) labeling solution (Invitrogen, C1157) was added to the isolated cells. The compounds of Examples were dissolved in DMSO to make a 5 mM stock, then treated to make 1.25 µM, 2.5 µM, 5 µM, and 10 µM, respectively. After 5 days, the degree of cell proliferation was measured by the amount of CFSE staining using flow cytometry, and the results are shown in FIG. 2.

As shown in FIG. 2, as a result of treating CD4+ and CD8+ T cells with the compounds of Examples, it could be confirmed that cell proliferation is inhibited.

### Experimental Example 2: Inhibition of IL-17 factor in gdT cells

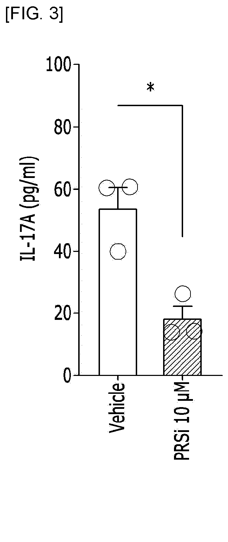

After collecting 10 ml of normal human blood, ficoll density gradient medium (Cytiva, 17144003) and PBS, the immune cell layer (PBMC) was isolated using a centrifuge. PBMCs were transferred to a new tube using a pipette. After RBC lysis buffer was added to PBMC, centrifugation was performed, and then 10 ng/ml of human IL-2 and IL-15 protein (cytokine) were respectively added to the cells, and cultured (for the purpose of inducing IL-17A). The compounds of Examples were dissolved in DMSO to make a 5 mM stock, and then 10 µM of the example compound was treated. On the 3rd and 5th days of cytokine treatment, 10 ng/ml of human IL-2 and IL-15 proteins (cytokine) were added, centrifuged, and the supernatant was collected. The IL-17A expression level was confirmed with the collected supernatant using an IL-17A ELISA kit (DY371, R&D Systems), and the results are shown in FIG. 3.

As shown in FIG. 3, when gdT cells isolated from normal human blood were treated with the compounds of Examples, it was confirmed that IL-17 cytokine, which is a key factor of gdT cells, was inhibited.

### Experimental Example 3: Improvement of renal function and anti-fibrotic efficacy - Mouse renal failure treatment model by Adenine diet

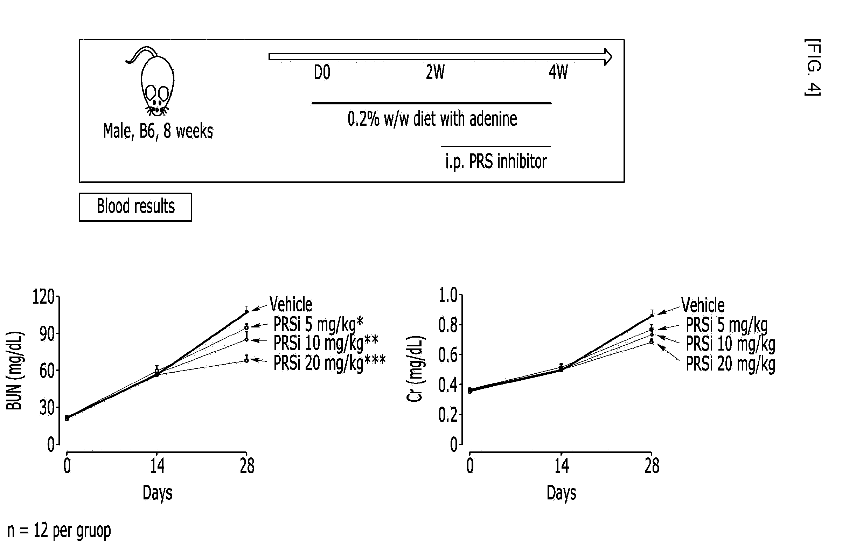

A feed containing adenine was administered to C57BL/6 male mice as described in Ali et al. J Pharmacol Toxicol Methods 2013; Jia et al. BMC Nephrol 2013; Tanaka et al. Am J Pathol 2009. Starting 2 weeks after the start of adenine administration, 5 mg/kg, 10 mg/kg and 20 mg/kg of the compounds of Examples were orally administered daily for 2 weeks (FIG. 4). Physiological saline was administered to the negative control group (vehicle). Blood BUN and Creatinine, which are the primary indicators of kidney function, were measured. The results are shown in FIG. 4.

As shown in FIG. 4, as a result of oral administration (once a day) of the compounds of Examples, it was confirmed that the deterioration of renal function was significantly inhibited in a dose-dependent manner.

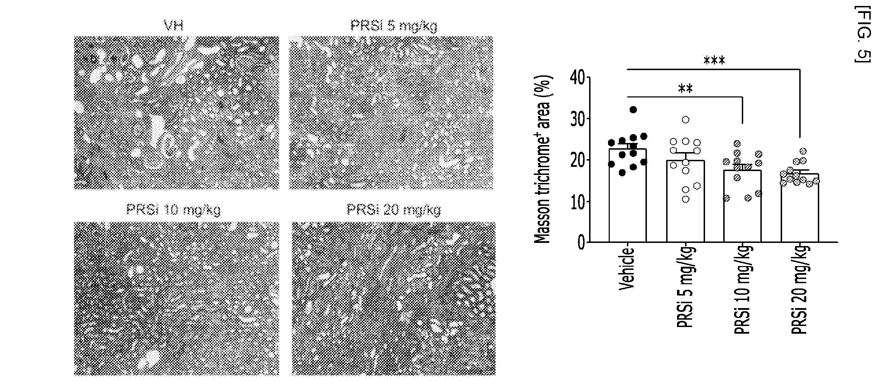

Further, the degree of fibrosis of the mouse kidney tissue was measured through immunochemical staining, and the results are shown in FIG. 5.

As shown in FIG. 5, it was confirmed that in a mouse renal failure model caused by an Adenine diet, it was confirmed that excessive accumulation of collagen in renal tissue was significantly inhibited in a dose-dependent manner following oral administration of the compounds of Examples.

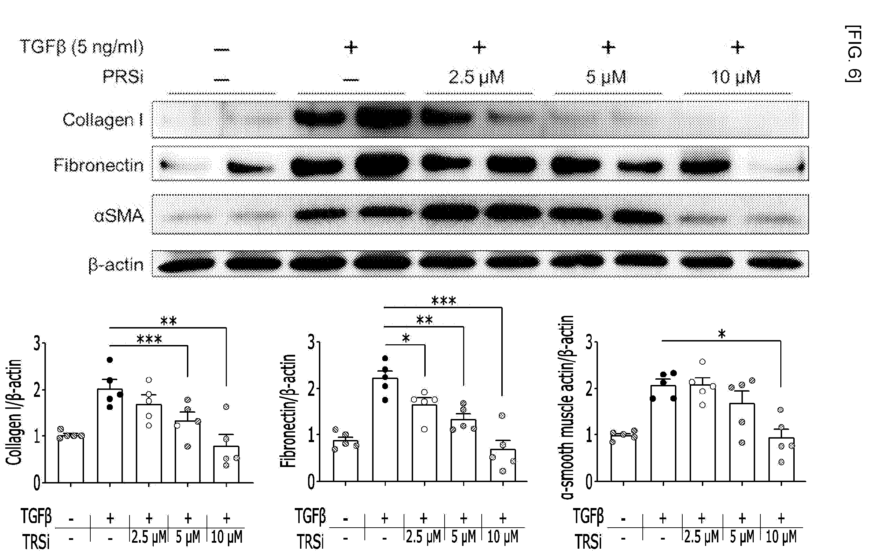

In addition, the mouse kidney tissue was crushed using a tissue homogenizer (TissueLyser II, Qiagen), centrifuged, and then the supernatant was collected. The supernatant was centrifuged, and the cells were isolated and cultured. The cultured cells were treated with 5 ng/ml TGFb and 2.5 µM, 5 µM, and 10 µM of the compounds of Examples, respectively, and 48 hours later, cells were harvested, and western blot was performed to quantitatively analyze fibrotic factor proteins. The results are shown in FIG. 6.

As shown in FIG. 6, Rat kidney cell line (NRK-49F cell line) was treated with the compounds of Examples, and as a result, it was confirmed that fibrotic factors (collagen, Fibronectin, αSMA) were significantly inhibited.

### Experimental Example 4: Fibrotic factor inhibition effect

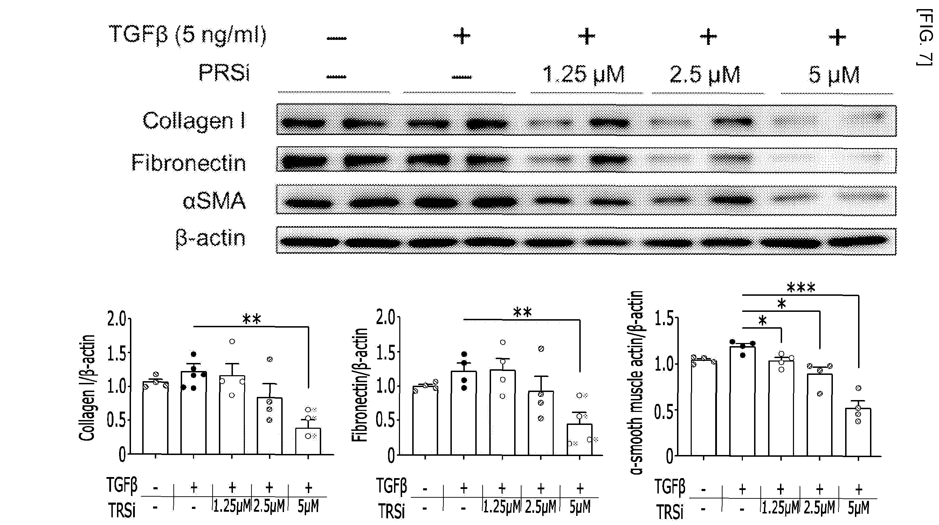

Digestion buffer was added to human kidney tissue (preserved after biopsy), cut into small pieces with scissors, and then disintegrated through a shaker incubation for 90 minutes. After filtering the disintegrated tissue through a 70 µm filter cell strainer, the remaining tissue was filtered once again using a 40 µm filter cell strainer. The filtered disintegrated tissue solution was centrifuged, and the cells were isolated and cultured. The cultured cells were treated with 5 ng/ml TGFb and 1.25 µM, 2.5 µM, and 5 µM of the compounds of Examples, respectively, and then 48 hours later, cells were harvested, and western blot was performed to quantitatively analyze fibrotic factor proteins. The results are shown in FIG. 7.

As shown in FIG. 7, as a result of treating human primary kidney cells with the compounds of Examples, it was confirmed that fibrotic factors (collagen, fibronectin, αSMA) were significantly inhibited.

### Experimental Example 5: Improvement of renal function and anti-fibrotic effect - Mouse renal failure prevention model by adenine diet

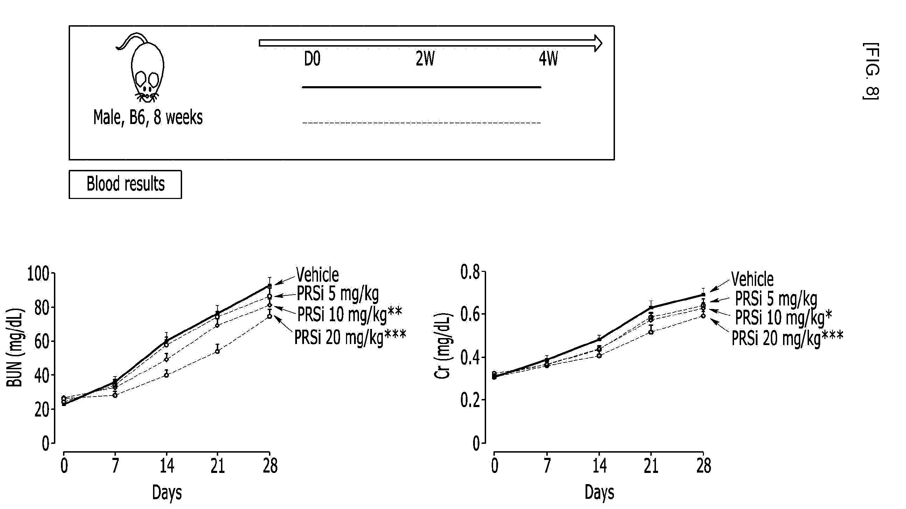

A feed containing adenine was administered to C57BL/6 male mice as described in Ali et al. J Pharmacol Toxicol Methods 2013; Jia et al. BMC Nephrol 2013; Tanaka et al. Am J Pathol 2009. Simultaneously with the start of adenine administration, 5 mg/kg, 10 mg/kg, and 20 mg/kg of the compounds of Examples were orally administered daily for 4 weeks (FIG. 8). Physiological saline was administered to the negative control group (vehicle). Blood BUN and Creatinine, which are the primary indicators of kidney function, were measured. The results are shown in FIG. 8 below.

As shown in FIG. 8, an Adenine diet was administrated to normal mice for 4 weeks and, at the same time, the compound of Examples was orally administered (once a day) for 4 weeks, and as a result, it was confirmed that the deterioration of renal function was significantly inhibited in a dose-dependent manner. That is, it exhibited preventive effect by suppressing kidney damage caused by Adenine diet.

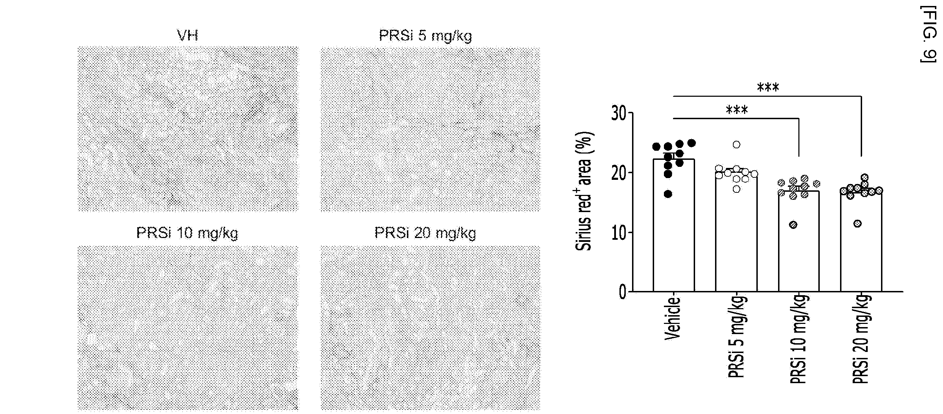

Additionally, the degree of fibrosis of the mouse kidney tissue was measured through immunochemical staining. The results are shown in FIG. 9. As shown in FIG. 9, it was confirmed that as the compounds of Examples were simultaneously administered orally in a mouse renal failure model induced by an Adenine diet, excessive collagen accumulation in renal tissue was significantly inhibited in a dose-dependent manner.

### Experimental Example 6: Improvement of renal function and anti-fibrotic efficacy - NTN (Nephrotoxic serum-induced Nephritis) model

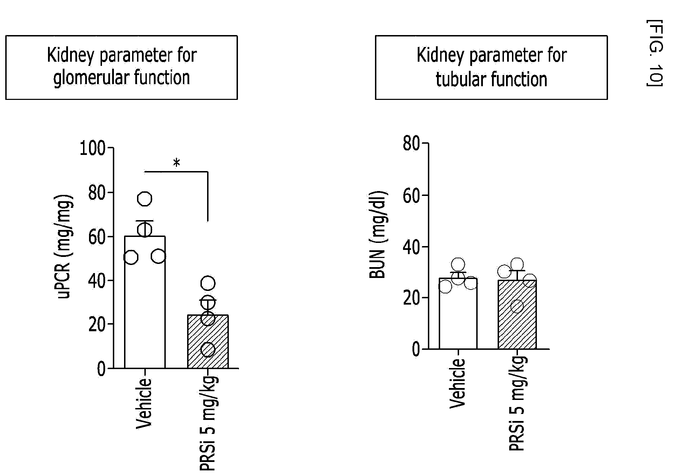

After sensitizing C57BL/6 male mice with 200 ug sheep IgG and Freund's complement adjuvant, 10 µg of sheep nephrotoxic serum was injected after five days to induce disease. From the day the disease was induced, the compounds of Examples were orally administered at doses of 5 mg/kg, 10 mg/kg, and 20 mg/kg for 7 days, respectively. Saline was administered to the negative control group (vehicle). On day 8, renal function (uPCR, BUN) was assessed. The results are shown in FIG. 10.

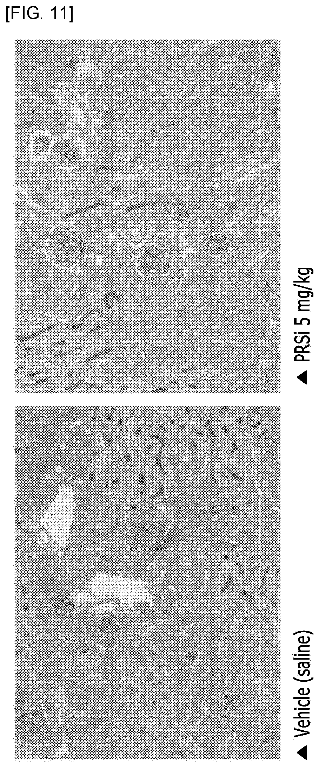

The NTN model is a glomerulonephritis model that causes inflammation in the glomeruli, which induces proteinuria. As shown in FIG. 10, it can be confirmed that this inflammation occurred specifically in the glomeruli (left vehicle in FIG. 10), and almost no damage to the tubules occurred (right vehicle in FIG. 10). As a result of oral administration of the compounds of Examples for one week, it was found to be effective in damaged glomerular tissue by significantly reducing proteinuria (PRSi 5 mg/kg on the left side of FIG. 10, and FIG. 11).

## Claims

1. A pharmaceutical composition for preventing or treating nephritis, comprising a compound represented by the following Chemical Formula 1, or a pharmaceutically acceptable salt thereof:

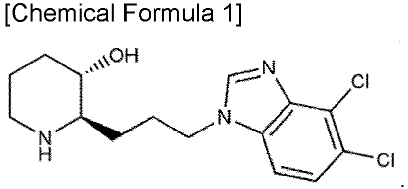

2. The pharmaceutical composition of claim 1, wherein
the nephritis is tubulointerstitial nephritis, glomerulonephritis, acute pyelonephritis, or chronic pyelonephritis.

3. The pharmaceutical composition of claim 1, wherein
the pharmaceutically acceptable salt is hydrochloride.

4. The pharmaceutical composition of claim 1, wherein
the pharmaceutical composition has an effect of improving renal function.

5. The pharmaceutical composition of claim 1, wherein
the pharmaceutical composition has an effect of reducing inflammatory cells CD4+, CD8+ or gdT in renal tissue.
